# EUROPEAN PATENT APPLICATION

(11) **EP 4 721 771 A1**
(43) Date of publication of application: **08.04.2026**
(21) Application number: 24815271.2
(22) Date of filing: 20.05.2024
(51) Int. Cl.: A61L 27/26, A61L 27/38, A61L 27/44, A61L 27/52, C08L 71/02, C12N 5/07

(54) **HYDROGEL CAPABLE OF INSTANTLY SOLIDIFYING**

(30) Priority: 26.05.2023 JP 2023086729
(71) Applicant: The University of Tokyo, Bunkyo-ku, Tokyo 113-8654 (JP); Gellycle Co., Ltd., Tokyo 113-0033 (JP)
(72) Inventor: SAKAI Takamasa, Tokyo 113-8654 (JP); ISHIKAWA Shohei, Tokyo 113-8654 (JP); KAMATA Hiroyuki, Tokyo 113-0033 (JP)
(74) Representative: Symbiosis IP Limited
(86) International application number: PCT/JP2024/018504
(87) International publication number: WO 2024/247787

(57) **Abstract**

An object of the present invention is to provide a hydrogel material capable of being gelated in situ in a short time using a general-purpose means such as spraying and having cell compatibility and tissue adhesiveness.

It has been found that a material which solidifies in a short time and has cell compatibility and tissue adhesiveness can be provided by forming a hydrogel having two kinds of network structures: a first polymer network in which polymers of the same kind having a polyethylene glycol (PEG) backbone are crosslinked; and a second polymer network in which a polypeptide and a heterologous polymer called a PEG backbone polymer are crosslinked.

## Description

### Technical Field

The present invention relates to a tissue-adhesive hydrogel capable of instantly solidifying by spraying or the like, and a kit for forming the hydrogel.

### Background Art

A hydrogel is a polymeric material composed of a crosslinked polymer and water, and has high permeability to molecules necessary for living cells such as salts and proteins, and thus is used as a cell scaffold material. As the polymer, which constitutes a scaffold material, of such a hydrogel, naturally occurring collagen, gelatin, fibrinogen/thrombin, polysaccharides, or synthetic polymers such as poly(acrylamide) and poly(ethylene glycol) (PEG) can be used (for example, Non Patent Literature 1). Alginic acid is also present as a material that rapidly gelates, and in general, alginic acid gel does not exhibit cell adhesiveness and is not suitable as a scaffold material for cell adhesion.

Gelatin has been extensively studied as an artificial extracellular matrix (ECM) since its high water solubility and cell adhesion properties are suitable for cell-related manipulations (for example, Non Patent Literature 2). Gelatin is also characterized in that the risk of contamination by unknown viruses is minimized compared to other protein-derived products by applying strict chemical and physical treatments in the manufacturing process.

In order to gelate gelatin, a method of crosslinking lysine residues in gelatin by a synthetic polymer has been attempted, but conventionally, it has been difficult to form an ECM gel in situ since the gelation time requires about several minutes in a neutral pH region. In the case of such slow gelation, since a gelling solution flows out to the outside before being solidified, the hydrogel cannot be formed on the surface of the biological tissue having a complicated shape or an inclined surface. Therefore, development of a hydrogel material capable of gelating in a short time in a neutral pH environment, which is an ideal condition for living cells, and satisfying both cell compatibility and tissue-adhesive has not yet been achieved.

### Citation List

### Non Patent Literatures

Non Patent Literature 1: Asadi et al., Mater. Chem. Phys. 2020, 242, 122528.
Non Patent Literature 2: Su et al., Biotechnol. Lett. 2015, 37 (11), 2139-2145.

### Summary of Invention

### Technical Problem

Therefore, an object of the present invention is to provide a hydrogel material capable of being gelated in situ in a short time using a general-purpose means such as spraying and having cell compatibility and tissue adhesiveness.

### Solution to Problem

As a result of intensive studies to solve the above problems, the present inventors have found that a material which solidifies in a short time and has cell compatibility and tissue adhesiveness can be provided by forming a hydrogel having two kinds of network structures: a first polymer network in which polymers of the same kind having a polyethylene glycol (PEG) backbone are crosslinked; and a second polymer network in which a polypeptide and a heterologous polymer called a PEG backbone polymer are crosslinked. The present inventors have also found that when cells such as stem cells are supported (encapsulated) in such a hydrogel, the hydrogel can be suitably used as a scaffold material for cell adhesion, and a hydrogel composition including a desired cell can be effectively immobilized on a target biological tissue. Based on these findings, the present invention has been completed.

That is, in an aspect, the present invention relates to a hydrogel, a scaffold material for cell adhesion containing the hydrogel, and a gel composition including cells supported in the hydrogel and provides as follows:
<1> A hydrogel comprising: a first polymer network having a structure in which two or more polymers (PEG-A) having a polyethylene glycol backbone are crosslinked with each other; and a second polymer network having a structure in which a polypeptide and a polymer (PEG-B) having a polyethylene glycol backbone are crosslinked with each other;
<2> The hydrogel according to <1>, wherein the hydrogel has a gelation time within a range of 10 seconds, and wherein the gelation time is a time required until a storage elastic modulus G' and a loss elastic modulus G" satisfy G' = G";
<3> The hydrogel according to <1>, wherein a concentration of the polypeptide in the second polymer network is 10 g/L or more and 120 g/L or less in the hydrogel;
<4> The hydrogel according to <1>, wherein a total content of the polymers having a polyethylene glycol backbone (PEG-A and PEG-B) in the hydrogel is 15 g/L or more;
<5> The hydrogel according to <1>, wherein the first polymer network has a cleavable moiety selected from the group consisting of an ester bond, a thioester bond, a disulfide bond, a phosphate ester bond, a sulfonate ester bond, and a combination thereof;
<6> The hydrogel according to <1>, wherein the polymers (PEG-A) having a polyethylene glycol backbone in the first polymer network comprise a first polymer having two or more nucleophilic functional groups in total at a side chain or a terminal and a second polymer having two or more electrophilic functional groups in total at a side chain or at a terminal;
<7> The hydrogel according to <1>, wherein the polymer (PEG-B) having a polyethylene glycol backbone in the second polymer network has two or more electrophilic functional groups capable of reacting with an amino group present in a molecule of the polypeptide to form an amide bond or a urethane bond at a side chain or a terminal;
<8> The hydrogel according to <1>, wherein the polypeptide is one or more selected from the group consisting of gelatin, collagen, albumin, laminin, fibrinogen, elastin, fibronectin, or a derivative thereof;
<9> The hydrogel according to <1>, wherein a concentration of the polypeptide in the hydrogel is 10 g/L or more, and a concentration of the polymer (PEG-B) having a polyethylene glycol backbone in the second polymer network is 15 g/L or more;
<10> The hydrogel according to <1>, wherein a pH of a solvent contained in the hydrogel is in a range of 6.5 to 11.5;
<11> A scaffold material for cell adhesion, containing the hydrogel according to any one of <1> to <10>;
<12> A gel composition including cells supported in the hydrogel according to any one of <1> to <10>; and
<13> The gel composition according to <11>, wherein the cells are somatic stem cells.

In another aspect, the present invention relates to a kit including a raw material polymer solution for forming the hydrogel, and specifically, provides as follows:
<14> A kit for rapidly preparing a hydrogel, including: a solution X containing polymers A and B and a solution Y containing a polypeptide and a polymer C separately stored, wherein the polymer A is a polymer having a polyethylene glycol backbone chemically crosslinkable with the polypeptide, the polymers B and C are a combination of polymers having a polyethylene glycol backbone chemically crosslinkable with each other, and a gelation time of the hydrogel obtained by mixing the solutions X and Y is within 10 seconds, the gelation time being a time required until a storage elastic modulus G' and a loss elastic modulus G" satisfy G' = G";
<15> The kit according to <14>, wherein the polymer A has two or more electrophilic functional groups capable of reacting with an amino group present in a molecule of the polypeptide to form an amide bond or a urethane bond at a side chain or a terminal;
<16> The kit according to <14>, wherein the polymers B and C are a combination of a polymer having two or more nucleophilic functional groups in total at a side chain or a terminal and a polymer having two or more electrophilic functional groups in total at a side chain or a terminal;
<17> The kit according to <14>, wherein the polypeptide in the solution Y is one or more selected from the group consisting of gelatin, collagen, albumin, laminin, fibrinogen, elastin, fibronectin, or a derivative thereof;
<18> The kit according to <14>, wherein a reaction rate of the polymer B and the polymer C is in a range of 60% to 100%;
<19> The kit according to <14>, which is used for spraying the solutions X and Y onto a predetermined area by a sprayer; and
<20> The kit according to <14>, wherein the solutions X and Y are each stored in separate sprayers. Advantageous Effects of Invention

According to the present invention, it is possible to provide a novel hydrogel which solidifies in-situ in a short time and has cell compatibility and tissue adhesiveness. The hydrogel of the present invention typically solidifies almost instantaneously within a few seconds by being sprayed onto a surface of a living body, and maintains excellent cell adhesiveness derived from a polypeptide component in the gel.

By supporting cells such as stem cells in such a hydrogel, the hydrogel can be suitably used as a scaffold material for cell adhesion, and a desired cell can be effectively immobilized on a target biological tissue. Since the hydrogel solidifies almost instantaneously after being applied to the biological tissue by spraying or the like, the hydrogel does not flow out before gelation unlike a conventional gel material, and can be applied to an affected part having a complicated shape or an inclined surface. The hydrogel has protein permeability, and allows cells supported (encapsulated) in the gel to be stably survived for a long period of time.

### Brief Description of Drawings

Fig. 1(A) is a schematic view of a photograph and a structure of a hydrogel (GP) composed of gelatin and PEG-NHS; Fig. 1(B) is a graph showing gelation times of Comparative Examples 16, 17, and 18; and Fig. 1(C) is a graph showing changes over time in storage elastic modulus (G') and loss elastic modulus (G") of Comparative Example 17.
Fig. 2(A) is a schematic view of a photograph and a structure of a hydrogel (PP) composed of PEG-SH and PEG-MA; Fig. 2(B) is a graph showing gelation times of Reference Examples 1, 2, 3, and 4; Fig. 2(C) is a graph showing changes over time in storage elastic modulus (G') and loss elastic modulus (G") of Reference Example 3.
Fig. 3(A) is a schematic view of a photograph and a structure of a hydrogel (GP/PP) composed of Gelatin/PEG-SH and PEG-NHS/PEG-MA; and Fig. 3(B) is a graph showing changes over time in storage elastic modulus (G') and loss elastic modulus (G") of Example 3.
Fig. 4 is a graph showing elastic moduli (G') of hydrogels of Comparative Example 17, Reference Example 3, and Example 3.
Fig. 5 is an image showing a process of forming the hydrogel of Example 3 by a sprayer.
Fig. 6 shows a representative photograph (left figure) of GP/PP (ester) in which the hydrogel of Example 3 is made hydrolyzable in a collagenase solution and an alkaline aqueous solution, and a graph (right figure) showing the swelling degree (Q) of the hydrogel.
Fig. 7 shows a glass slide to which a collagen casing is fixed (upper left figure), a schematic view (upper right figure) of a chemical reaction at an interface between a GP hydrogel and the collagen casing, and a schematic view (lower figure) of a test piece subjected to a lap shear test.
Fig. 8 shows a representative photograph (left figure) of a test piece set in a tensile tester, a graph (center figure) showing a representative relationship between a lap shear strength and displacement, and a graph (right figure) showing a lap shear strength at fracture of each hydrogel.
Fig. 9(A) shows a device (left figure) for simultaneously spraying human mesenchymal stem cells (hMSCs) and a hydrogel component, and fluorescence images of hMSCs encapsulated in the hydrogel (right figure: scale bar indicates 100 µm); and Fig. 9(B) is a graph showing viability of hMSCs cultured in each hydrogel.
Fig. 10 shows an experimental procedure in which bovine serum albumin (BSA-FITC) labeled with fluorescein isothiocyanate was added to a culture solution (left figure); and fluorescence images of hMSC cultured in BSA-FITC (right figure: 200× and 400× indicate magnification ratios; and scale bar indicates 40 µm).

### Description of Embodiments

Hereinafter, embodiments of the present invention will be described. The scope of the present invention is not limited by these descriptions and can be embodied with modifications as appropriate other than the following exemplary embodiments but not departing from the gist of the present invention.

### 1. Hydrogel of Present Invention

A hydrogel of the present invention is characterized by having the following two network structures:
i) a first polymer network formed by chemically crosslinking two or more polymers (PEG-A) having a polyethylene glycol (PEG) backbone with each other; and
ii) a second polymer network formed by chemically crosslinking a polypeptide and a polymer (PEG-B) having a polyethylene glycol backbone.

That is, the first polymer network is a network structure formed by crosslinking polymers having a PEG backbone with each other, and the second polymer network is a network structure formed by crosslinking a polypeptide molecule and a heterologous polymer called a PEG polymer. As a result, reduction in gelation time and solidification in a short time, which are problems of the prior art, have been achieved while maintaining cell compatibility and tissue adhesiveness derived from the polypeptide component.

In the present specification, the "gel" is generally a dispersion system of a polymer that has high viscosity and lost fluidity, refers to a state having a relationship of G' ≥ G" between a storage elastic modulus G' and a loss elastic modulus G", and is typically a substance having a three-dimensional network structure. The hydrogel refers to a state in which the gel contains a solvent such as water. Preferably, the solvent is water.

The hydrogel of the present invention has a gelation time preferably within 10 seconds, more preferably in a range of 1 to 10 seconds. Since the hydrogel of the present invention gelates in a short time and solidifies almost instantaneously, a solution before gelation does not flow out to the outside or a peripheral portion unlike a conventional gel material, and the hydrogel can be applied to an affected part having a complicated shape or an inclined surface. Here, the "gelation time" is a time from mixing of a solution containing a raw material polymer to gelation, and is a time required until the storage elastic modulus G' and the loss elastic modulus G" satisfy G' = G". Such a gelation time can be further adjusted mainly by appropriately setting the polymer concentration, pH, and ionic strength in the polymer solution.

As described above, the solvent contained in the hydrogel of the present invention is typically water, but in some cases, may be a mixed solvent containing a trace amount of alcohols such as ethanol and other organic solvents. The pH of the solvent contained in the hydrogel is in a range of 6.5 to 11.5, more preferably in a range of 7.0 to 10.5, and preferably in a range of 7.0 to 8.5.

### 1-1 PEG Polymer

Both PEG-A and PEG-B in the hydrogel of the present invention are polymers having a polyethylene glycol backbone. The "polymer having a polyethylene glycol backbone" (hereinafter, simply referred to as "PEG polymer" in some cases) typically includes polymer species having a plurality of branches of a polyethylene glycol backbone, and for example, a bi-, tri-, tetra-, or octa-branched polyethylene glycol is preferable. In particular, a gel including a tetra-branched polyethylene glycol backbone is known as a Tetra-PEG gel, and a network-structure network is constructed by an AB-type cross-end coupling reaction between two types of tetra-branched polymers having an electrophilic functional group such as an active ester structure and a nucleophilic functional group such as an amino group at each terminal (Matsunaga et al., Macromolecules, Vol. 42, No. 4, pp. 1344-1351, 2009). The Tetra-PEG gel can be easily prepared in situ by simple two-liquid mixing of each polymer solution, and the gelation time can also be controlled by adjusting the pH and ionic strength at the time of gel preparation. The gel containing PEG as a main component is also excellent in biocompatibility.

In the first polymer network, two or more kinds of PEG polymers (PEG-A) are chemically crosslinked with each other to form a three-dimensional network structure. Typically, the PEG polymer (PEG-A) forming the first polymer network includes a first polymer having a total of two or more nucleophilic functional groups at a side chain or a terminal and a second polymer having a total of two or more electrophilic functional groups at a side chain or a terminal. The nucleophilic functional group and the electrophilic functional group are crosslinked to form a gel. The total of the nucleophilic functional group and the electrophilic functional group is preferably 5 or more. These functional groups are still more preferably present at a terminal.

Examples of the nucleophilic functional group include a thiol group (a sulfhydryl group) (-SH), and an amino group, and those skilled in the art can appropriately use a known nucleophilic functional group. Preferably, the nucleophilic functional group is a -SH group. The nucleophilic functional groups may be the same as or different from each other, but are preferably the same. When the functional groups are the same, reactivity with an electrophilic functional group that forms a crosslinking bond becomes uniform, and a gel having a homogeneous three-dimensional structure is easily obtained.

As the electrophilic functional group, a functional group capable of being attacked by a nucleophilic functional group can be used. Examples of such a functional group include a maleimide group, an N-hydroxy-succinimidyl (NHS) group, a succinimidyl carbonate group, a sulfosuccinimidyl group, a phthalimidyl group, an imidazoyl group, an acryloyl group, a nitrophenyl group, and - CO₂PhNO₂ (Ph represents an o-, m-, or p-phenylene group), and those skilled in the art can appropriately use other known electrophilic functional groups. Preferably, the electrophilic functional group is a maleimide group, an N-hydroxysuccinimidyl (NHS) group, or a succinimidyl carbonate group. When the nucleophilic functional group is a thiol group, a maleimide group is particularly preferable as an electrophilic functional group, and when the nucleophilic functional group is an amino group, an N-hydroxysuccinimidyl (NHS) group or a succinimidyl carbonate group is particularly preferable as an electrophilic functional group. The electrophilic functional groups may be the same as or different from each other, but are preferably the same. When the functional groups are the same, reactivity with a nucleophilic functional group that forms a crosslinking bond becomes uniform, and a gel having a homogeneous three-dimensional structure is easily obtained.

A method for forming a crosslink for hydrogel formation is not limited to the reaction between the nucleophilic functional group and the electrophilic functional group described above, and those skilled in the art can select other known chemically crosslinkable pairs.

In a preferred embodiment, the first polymer network can have a bond that can be resolved by hydrolysis or the like. That is, the first polymer network can have a cleavable moiety selected from the group consisting of an ester bond, a thioester bond, a disulfide bond, a phosphate ester bond, a sulfonate ester bond, and a combination thereof. The cleavable moiety can be a linker moiety linking the polyethylene glycol backbone to the nucleophilic/electrophilic functional groups described above. Alternatively, the cleavable moiety can be present at a crosslinking position between PEG-As.

The PEG polymer (PEG-B) forming the second polymer network is also similar to the PEG-A in that it has a polyethylene glycol backbone. The PEG-B forms the second polymer network by chemically crosslinking with a polypeptide. Therefore, typically, the PEG-B has a total of two or more electrophilic functional groups capable of reacting with an amino group present in a molecule of the polypeptide to form an amide bond or a urethane bond at a side chain or a terminal. The kind of such an electrophilic functional group is as described above.

The PEG-A and the PEG-B each independently have a weight average molecular weight (Mw) in a range of 1x10³ to 1x10⁵, preferably in a range of 0.5x10⁴ to 5x10⁴, and more preferably in a range of 1x10⁴ to 2x10⁴.

Preferred non-limiting specific examples of the PEG polymer having a nucleophilic functional group at a terminal (that is, the first polymer of PEG-A) include a compound represented by the following Formula (I) having four branches of a polyethylene glycol backbone and having a thiol group at a terminal.

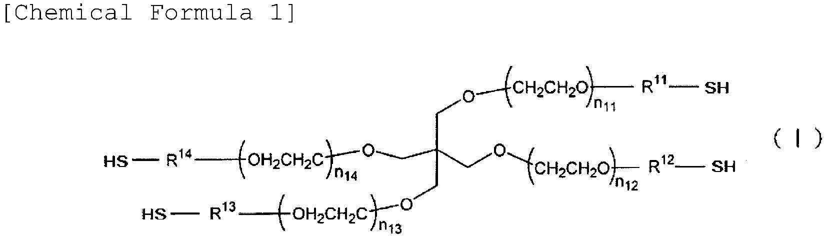

In Formula (I), R¹¹ to R¹⁴ are the same as or different from each other and each represent a C₁-C₇ alkylene group, a C₂-C₇ alkenylene group, -NH-R¹⁵-, -CO-R¹⁵-, -R¹⁶-O-R¹⁷-, -R¹⁶-NH-R¹⁷-, -R¹⁶-CO₂-R¹⁷-, -R¹⁶-CO₂-NH-R¹⁷-, -R¹⁶-CO-R¹⁷-, or -R¹⁶-CO-NH-R¹⁷-, where R¹⁵ represents a C₁-C₇ alkylene group, R¹⁶ represents a C₁-C₃ alkylene group, and R¹⁷ represents a C₁-C₅ alkylene group.

n₁₁ to n₁₄ may be the same as or different from each other. As the values of n₁₁ to n₁₄ are closer to each other, a homogeneous three-dimensional structure can be obtained, and the strength becomes higher. Therefore, in order to obtain a high-strength gel, n₁₁ to n₁₄ are preferably the same. When the values of n₁₁ to n₁₄ are too high, the strength of the gel is weak, and when the values of n₁₁ to n₁₄ are too low, the gel is hardly formed due to steric hindrance of the compound. Therefore, n₁₁ to n₁₄ are, for example, an integer value of 5 to 600, preferably 25 to 250, more preferably 50 to 120, and still more preferably 110 to 120.

In the above Formula (I), R¹¹ to R¹⁴ are linker moieties connecting a functional group and a core moiety. R¹¹ to R¹⁴ may be the same as or different from each other, but are preferably the same in order to produce a high-strength gel having a homogeneous three-dimensional structure. R¹¹ to R¹⁴ represent a C₁-C₇ alkylene group, a C₂-C₇ alkenylene group, -NH-R¹⁵-, -CO-R¹⁵-, -R¹⁶-O-R¹⁷-, -R¹⁶-NH-R¹⁷-, -R¹⁶-CO₂-R¹⁷-, -R¹⁶-CO₂-NH-R¹⁷-, -R¹⁶-CO-R¹⁷-, or -R¹⁶-CO-NH-R¹⁷-. R¹⁵ represents a C₁-C₇ alkylene group. R¹⁶ represents a C₁-C₃ alkylene group. R¹⁷ represents a C₁-C₅ alkylene group.

The "C₁-C₇ alkylene group" means an optionally branched alkylene group having 1 or more and 7 or less carbon atoms, and means a linear C₁-C₇ alkylene group or a C₂-C₇ alkylene group having one or two or more branches (the number of carbon atoms including the branch is 2 or more and 7 or less). Examples of the C₁-C₇ alkylene group include a methylene group, an ethylene group, a propylene group, and a butylene group. Examples of the C₁-C₇ alkylene group include -CH₂-, -(CH₂)₂-, -(CH₂)₃-, -CH(CH₃)**-,** -(CH₂)₃-, -(CH(CH₃))₂-, -(CH₂)₂-CH(CH₃)-, -(CH₂)₃-CH(CH₃)-, -(CH₂)₂-CH(C₂H₅)-, -(CH₂)₆-, -(CH₂)₂-C(C₂H₅)₂-, and -(CH₂)₃C(CH₃)₂CH₂-.

The "C₂-C₇ alkenylene group" is a linear or branched alkenylene group having 2 to 7 carbon atoms having one or two or more double bonds in the chain, and examples thereof include divalent groups having double bonds formed by eliminating 2 to 5 hydrogen atoms of adjacent carbon atoms from the alkylene group.

On the other hand, preferred non-limiting specific examples of the polymer having an electrophilic functional group at a terminal (that is, the second polymer in PEG-A; or PEG-B) include a compound represented by the following Formula (II) having four branches of a polyethylene glycol backbone and having an N-hydroxy-succinimidyl (NHS) group at a terminal.

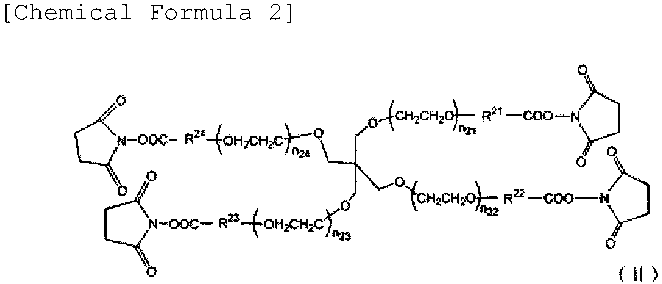

In the above Formula (II), n₂₁ to n₂₄ may be the same as or different from each other. As the values of n₂₁ to n₂₄ are closer, the gel can have a homogeneous three-dimensional structure and has a high strength, and thus it is preferable that the values are the same. When the values of n₂₁ to n₂₄ are too high, the strength of the gel is weak, and when the values of n₂₁ to n₂₄ are too low, the gel is hardly formed due to steric hindrance of the compound. Therefore, n₂₁ to n₂₄ are, for example, an integer value of 5 to 600, preferably 25 to 250, more preferably 50 to 120, and still more preferably 110 to 120.

In the above Formula (II), R²¹ to R²⁴ are linker moieties connecting a functional group and a core moiety. R²¹ to R²⁴ may be the same as or different from each other, but are preferably the same in order to produce a high-strength gel having a homogeneous three-dimensional structure. In Formula (II), R²¹ to R²⁴ may be the same as or different from each other, and represent a C₁-C₇ alkylene group, a C₂-C₇ alkenylene group, -NH-R²⁵-, -CO-R²⁵-, -R²⁶-O-R²⁷-, -R²⁶-NH-R²⁷-, -R²⁶-CO₂-R²⁷-, -R²⁶-CO₂-NH-R²⁷-, -R²⁶-CO-R²⁷-, or -R²⁶-CO-NH-R²⁷-. R²⁵ represents a C₁-C₇ alkylene group. R²⁶ represents a C₁-C₃ alkylene group. R²⁷ represents a C₁-C₅ alkylene group.

In the present specification, the alkylene group and the alkenylene group may have one or more arbitrary substituents. Examples of the substituent include, but are not limited to, an alkoxy group, a halogen atom (may be any of a fluorine atom, a chlorine atom, a bromine atom, and an iodine atom), an amino group, a mono- or disubstituted amino group, a substituted silyl group, an acyl group, and an aryl group. When the alkyl group has two or more substituents, these substituents may be the same or different. The same applies to alkyl moieties of other substituents including alkyl moieties (for example, alkyloxy groups, aralkyl groups, and the like).

In the present specification, when a certain functional group is defined as "optionally substituted", the type of substituent, substitution position, and the number of substituents are not particularly limited, and when there are two or more substituents, these substituents may be the same or different. Examples of the substituent include, but are not limited to, an alkyl group, an alkoxy group, a hydroxyl group, a carboxyl group, a halogen atom, a sulfo group, an amino group, an alkoxycarbonyl group, and an oxo group. Substituents may be further present in these substituents.

The content of PEG-A in the hydrogel is preferably 10 g/L or more and more preferably 20 g/L. The content of PEG-B in the hydrogel is preferably 5 g/L or more and more preferably 15 g/L or more.

Therefore, the total content of PEG-A and PEG-B in the hydrogel is preferably 15 g/L or more, more preferably 25 g/L or more, and still more preferably 35 g/L or more.

### 1-2 Polypeptide

As described above, the hydrogel of the present invention contains a polypeptide for forming the second polymer network by chemical crosslinking with the PEG-B. Typically, an amino group present in the molecule of the polypeptide reacts with an electrophilic functional group present at the side chain or terminal of the PEG-B to form a bond, whereby the polypeptide and the PEG-B are chemically crosslinked. The polypeptide imparts cell adhesiveness and the like to the hydrogel.

The polypeptide can be a natural product or an artificial product derived from a living body, and examples thereof include one or more selected from the group consisting of gelatin, collagen, albumin, laminin, fibrinogen, elastin, fibronectin, or a derivative thereof. These include a collagen peptide which is a protein obtained by hydrolyzing collagen, a substance obtained by chemically modifying collagen, and the like. Preferably, the polypeptide is gelatin or a derivative thereof.

The concentration of the polypeptide in the second polymer network is preferably 10 g/L or more and can be in a range of 120 g/L or less in the hydrogel.

Regarding the ratio of the polypeptide and the PEG-B in the second polymer network, in a preferred embodiment, the concentration of the polypeptide in the entire hydrogel is 10 g/L or more, and the concentration of the PEG-B is 15 g/L or more, and in a more preferred embodiment, the concentration of the polypeptide in the entire hydrogel is 20 g/L or more, and the concentration of the PEG-B is 30 g/L or more.

### 2. Gel Composition

In another aspect, the present invention relates to a scaffold material for cell adhesion containing the above-described hydrogel. As shown in Examples described later, the hydrogel of the present invention has excellent protein permeability, and cells supported (encapsulated) in the gel can stably survive for a long period of time.

The present invention also relates to a gel composition including celled supported (encapsulated) in the hydrogel. Such a gel composition is useful, for example, for effectively immobilizing a desired cell on a target biological tissue. As described above, since the hydrogel of the present invention has a very short gelation time and solidifies almost instantaneously after a raw material polymer solution is applied to a biological tissue by spraying or the like, the hydrogel does not flow out before gelation unlike a conventional gel material, and can be applied to an affected part having a complicated shape or an inclined surface.

The cells included in the gel composition of the present invention are not particularly limited, and a wide variety of cells can be used. The type and the like of the cells are not particularly limited, and can be appropriately selected according to the purpose, and can be used for all cells, taxonomically, for example, regardless of eukaryotic cells, prokaryotic cells, multicellular biological cells, and unicellular biological cells. These may be used singly or in combination of two or more kinds thereof.

Examples of the eukaryotic cells include animal cells, insect cells, plant cells, and fungi. These may be used singly or in combination of two or more kinds thereof. Adherent cells are not particularly limited, and can be appropriately selected according to the purpose, and examples thereof include differentiated cells and undifferentiated cells. These may be used singly or in combination of two or more kinds thereof. Examples of the differentiated cells include somatic stem cells and hepatocytes which are liver parenchymal cells; stellate cells; Kupffer cells; vascular endothelial cells; endothelial cells such as sinusoidal endothelial cells and corneal endothelial cells; fibroblasts; osteoblasts; osteoclasts; periodontal ligament-derived cells; epidermal cells such as epidermal keratinocytes; tracheal epithelial cells; intestinal epithelial cells; cervical epithelial cells; epithelial cells such as corneal epithelial cells; mammary glandular cells; pericytes; muscle cells such as smooth muscle cells and myocardial cells; renal cells; pancreatic islet cells; nerve cells such as peripheral nerve cells and optic nerve cells; chondrocytes; and bone cells. The adherent cells may be primary cells directly taken from tissues or organs, or may be cells after several passages. These may be used singly or in combination of two or more kinds thereof. The undifferentiated cells are not particularly limited, and can be appropriately selected according to the purpose, and examples thereof include pluripotent stem cells such as embryonic stem cells, which are undifferentiated cells, and mesenchymal stem cells having pluripotency; unipotent stem cells such as vascular endothelial progenitor cells having unipotency; and induced pluripotent stem cells (iPS cells). These may be used singly or in combination of two or more kinds thereof. Examples of the prokaryotic cells include eubacteria and archaea. Somatic stem cells are preferable.

### 3. Kit

The present invention also further relates to a kit for rapidly preparing the above-described hydrogel.

The kit of the present invention separately stores a solution X containing polymers A and B and a solution Y containing a polypeptide and a polymer C. That is, these solutions X and Y separately store the polymers B and C, which are PEG polymers forming the first polymer network and the polypeptide and the polymer A which form the second polymer network. By mixing these solutions X and Y, crosslinking between the polymers proceeds, and a hydrogel having these polymer networks is formed.

More specifically, in the kit of the present invention,
· the polymer A is a polymer having a polyethylene glycol backbone chemically crosslinkable with the polypeptide;
· the polymers B and C are a combination of polymers having a polyethylene glycol backbone chemically crosslinkable with each other; and
· a gelation time of the hydrogel obtained by mixing the solutions A and B is within 10 seconds, preferably in a range of 1 to 10 seconds, the gelation time being a time required until a storage elastic modulus G' and a loss elastic modulus G" satisfy G' = G".

The polymers B and C correspond to the first polymer and the second polymer in the PEG-A described above, and preferred aspects thereof are as described above.

The polymer A corresponds to the PEG-B described above. The polypeptide contained in the solution Y corresponds to the polypeptide forming the second polymer network described above. These preferred embodiments are also as described above.

The concentration of the polymer A in the solution X is in a range of 10 to 240 g/L, preferably in a range of 20 to 120 g/L, and more preferably 30 to 90 g/L. The concentration of the polypeptide in the solution Y is in a range of 20 to 120 g/L, preferably in a range of 20 to 80 g/L, and more preferably in a range of 20 to 60 g/L.

The concentrations of the polymer B and the polymer C in the solutions X and Y are each in a range of 10 to 240 g/L, preferably in a range of 20 to 150 g/L, more preferably in a range of 20 to 100 g/L, and still more preferably in a range of 20 to 60 g/L. The concentrations of the polymers B and C may be the same or different as long as the above range is satisfied, but are preferably the same.

The solutions X and Y are adjusted so that the pH of a mixed solution obtained by mixing these solutions is in a region acceptable to cells (pH 5.3 or more), and is adjusted so that the pH is preferably in a range of 6.5 to 11.5, more preferably in a range of 7.0 to 10.5, and still more preferably in a range of 7.0 to 8.5. In a typical aspect, it is preferable that both solutions X and Y are within these pH ranges.

As the pH of the solutions X and Y, a pH buffer known in the art can be used. For example, the pH can be adjusted to the above range by using a citric acid-phosphate buffer (CPB) and changing the mixing ratio of citric acid and disodium hydrogen phosphate.

The solvent in the solutions X and Y is water, but in some cases, may be a mixed solvent containing alcohols such as ethanol and other organic solvents. Preferably, the solutions X and Y are aqueous solutions using water as a sole solvent.

The volumes of the solutions X and Y in the kit of the present invention can be appropriately adjusted according to the area of the biological tissue or site to which they are applied, the complexity of the structure, and the like, and are typically in a range of 0.1 to 20 mL and preferably 1 to 10 mL.

In a preferred aspect, the reaction rate of the polymer B and the polymer C is in a range of 60% to 100%. Within such a range, a desired gelation time and the like can be adjusted.

As a means for mixing the solutions X and Y by dropwise addition, for example, a two-liquid mixing syringe as disclosed in WO 2007/083522 A1 can be used. The temperature of the two liquids at the time of mixing is not particularly limited as long as each polymer is dissolved and each liquid has fluidity. For example, the temperatures of the two liquids may be different, but it is preferable that the temperatures are the same because the two liquids are easily mixed.

The kit of the present invention may include a means for spraying the solutions X and Y to a predetermined area by a sprayer. As such a means, a sprayer in which the solutions X and Y are separately stored can be used. As the sprayer, a known sprayer in the art can be appropriately used, and a medical sprayer is preferable. Therefore, such a sprayer can be used as a container in the kit of the present invention, and in this case, one aspect of the present invention can be a medical device, preferably a sprayer, storing the solutions X and Y.

### Examples

Hereinafter, the present invention will be described in more detail with reference to Examples; however, the present invention is not limited thereto.

### Materials:

Porcine skin-derived gelatin (APAT, M_{w} = 60 kg/mol) and bovine skin-derived collagen casing were purchased from Nippi Inc. (Tokyo, Japan). N-Hydroxysuccinimide (NHS), maleimide (MA), or tetra-branched polyethylene glycol terminally-functionalized with a thiol (SH) group (M_{w} = 10 kg/mol) (PEG) (PEG-NHS, PEG-MA, and PEG-SH, respectively) was purchased from SINOPEG Biotech Co., Ltd. (Fujian Province, China). D-PBS(-) (PBS), Dulbecco's Modified Eagle Medium (DMEM), penicillin/streptomycin (PS), trypsin-EDTA (0.05%), fetal bovine serum (FBS), LIVE/DEAD (registered trademark) viability/cytotoxicity Kit, Calcein Blue AM, -Cellstain^{R}-PI solution, and fluorescein isothiocyanate-conjugated bovine serum albumin (FITC-BSA) were purchased from Thermo Fisher Scientific Inc. (Waltham, MA, USA). Clostridium histolyticum-derived collagenase was purchased from Sigma-Aldrich (St. Louis, MO, USA). Sodium hydroxide (NaOH) was purchased from FUJIFILM Wako Pure Chemical Corporation (Tokyo, Japan). Blue ink for visualizing a hydrogel component (Waterman Mystery Blue Ink) was purchased from The Waterman Pen Company (New York, NY, USA). A cyanoacrylate adhesive was purchased from Toagosei Co., Ltd. (Tokyo, Japan).

### Apparatus:

A 0.22 µm filter (Minisart, RC15-AC, Sartorius AG, Göttingen, Germany), a rheometer (MCR 301, Anton Paar GmbH, North Ryde, Australia), a tensile tester (Autograph AG-X plus, SHIMADZU CORPORATION, Kyoto, Japan), a fluorescence microscope (BZ-X710, Keyence Corp., Osaka, Japan), and a confocal laser scanning microscope (CLSM, LSM 800, Carl Zeiss AG, Jena, Germany) were used for the experiment.

### 1. Preparation of Polymer Solution and Gelation Step

As the polymer solutions A and B, polymer components having concentrations shown in Table 1 below were dissolved in PBS to prepare various polymer solutions. The polymer solution A is a solution containing gelatin and tetra-branched polyethylene glycol having an SH group at a terminal (PEG-SH), and the polymer solution B is a solution containing tetra-branched polyethylene glycol having an N-hydroxysuccinimide group at a terminal (PEG-NHS) and tetra-branched polyethylene glycol having a maleimide group at a terminal (PEG-MA). Here, gelatin and PEG-NHS (these are referred to as "GP pair") can be crosslinked with each other to form a hydrogel (GP gel), and PEG-SH and PEG-MA (these are referred to as "PP pair") can also be crosslinked with each other to form another hydrogel (PP gel).

**[Table 1]**

| | **Gelatin in A solution [g/L]** | **PEG-SH in A solution [g/L]** | **PEG-NHS in B solution [g/L]** | **PEG-MA in B solution [g/L]** | **Gelation time (*t_{gel}*)** | **Total content of PEG in hydrogel [g/L]** | |
|---|---|---|---|---|---|---|---|
| Comparative Example 1 | 120 | 60 | 180 | 60 | Unmeasurable | 150 | Gelatin is solidified at room temperature but is not gelated |
| Example 1 | 80 | 40 | 120 | 40 | Within 1 second | 100 | Cloudy after mixing |
| Example 2 | 60 | 30 | 90 | 30 | Within 1 second | 75 | Cloudy after mixing |
| Example 3 | 40 | 20 | 60 | 20 | 1 second | 50 | |
| Example 4 | 20 | 10 | 30 | 10 | 3 seconds | 25 | |
| Comparative Example 2 | 10 | 5 | 15 | 5 | Not gelated | 12.5 | |
| Example 5 | 40 | 50 | 60 | 50 | Within 1 second | 80 | |
| Example 6 | 40 | 100 | 60 | 100 | Within 1 second | 130 | |
| Example 7 | 40 | 150 | 60 | 150 | Within 1 second | 180 | |
| Example 8 | 40 | 200 | 60 | 200 | 1 second | 230 | |
| Comparative Example 3 | 140 | 20 | 210 | 20 | Unmeasurable | 125 | Gelatin is solidified at room temperature but is not gelated |
| Example 9 | 80 | 20 | 120 | 20 | 4 seconds | 80 | |
| Example 10 | 60 | 20 | 90 | 20 | 3 seconds | 65 | |
| Example 11 | 20 | 20 | 30 | 20 | 2 seconds | 35 | |
| Comparative Example 4 | 40 | - | 5 | - | Not gelated | 2.5 | |
| Comparative Example 5 | 40 | - | 10 | - | 8 minutes | 5 | |
| Comparative Example 6 | 40 | - | 20 | - | 4 minutes and 30 seconds | 10 | |
| Comparative Example 7 | 40 | - | 60 | - | 3 minutes | 30 | |
| Comparative Example 8 | 40 | - | 80 | - | 2 minutes and 45 seconds | 40 | |
| Comparative Example 9 | 40 | - | 120 | - | 2 minutes and 50 seconds | 60 | Cloudy after mixing |
| Comparative Example 10 | 40 | - | 160 | - | 3 minutes and 30 seconds | 80 | Cloudy after mixing |
| Comparative Example 11 | 40 | - | 180 | - | 3 minutes | 90 | Cloudy after mixing |
| Comparative Example 12 | 40 | - | 200 | - | 4 minutes | 100 | Cloudy after mixing |
| Comparative Example 13 | 40 | - | 220 | - | 30 minutes | 110 | Cloudy after mixing |
| Comparative Example 14 | 40 | - | 240 | - | 38 minutes | 120 | Cloudy after mixing |
| Comparative Example 15 | 160 | - | 240 | - | 1 minute | 120 | Cloudy after mixing |
| Comparative Example 16 | 80 | - | 120 | - | 1 minute | 60 | Cloudy after mixing |
| Comparative Example 17 | 40 | - | 60 | - | 3 minutes | 30 | |
| Comparative Example 18 | 26.7 | - | 40 | - | Not gelated | 20 | |
| Reference Example 1 | | 5 | | 5 | Not gelated | 5 | |
| Reference Example 2 | | 10 | | 10 | 3 seconds | 10 | |
| Reference Example 3 | | 20 | | 20 | 1 second | 20 | |
| Reference Example 4 | | 40 | | 40 | Within 1 second | 40 | |

Two solutions of the prepared polymer solutions A and B were mixed at a volume ratio of 1 : 1, and a gelation test was performed. The mixed gelling solution was poured into a glass vial, the time point at which the fluidity was lost when the vial was tilted was taken as the gelling point, and the time required to reach this point was observed as the gelation time "t_{gel}".

As a result, as shown in Table 1, in Examples 1 to 11, a gel was formed in a short time of several seconds or less. On the other hand, in Comparative Examples 1 to 4 and 18, no gel was formed. In Comparative Examples 5 to 17, although gel formation was observed, it took 1 minute or more for gelation, and in some cases, cloudiness was observed after mixing.

### 2. Verification of Gel Formation Behavior

Next, the gel formation behavior when the polymer solutions A and B were mixed was verified.

### <Rheological Property Evaluation>

The storage elastic modulus (G') and the loss elastic modulus (G") were measured by the following procedure. The polymer solution A was placed on the bottom plate of the rheometer and the same amount of the polymer solution B was added. A measuring plate (PP25, diameter: 3 mm) placed on top of the rheometer was set at a position 0.3 mm from the bottom plate. As experimental parameters, a temperature of 25°C, a shear amplitude of 1%, and a vibration frequency of 1.0 Hz were used. Changes over time in storage elastic modulus (G') and loss elastic modulus (G") of the gelling solution were measured.

### <Shear Elastic Modulus>

The shear elastic modulus was measured by the following procedure. The polymer solution A was poured into a silicone mold (diameter: 35 mm, height: 1 mm) and mixed with an equal amount of the polymer solution B. After incubation at 25°C for 24 hours, the formed hydrogel was gently removed from the mold and placed on the bottom plate of the rheometer. The measuring plate (PP25) was placed near the sample until a force of 1.5 N was detected. G' was measured at a strain of 0.1 to 10% at 25°C and an angular frequency of 10 Hz.

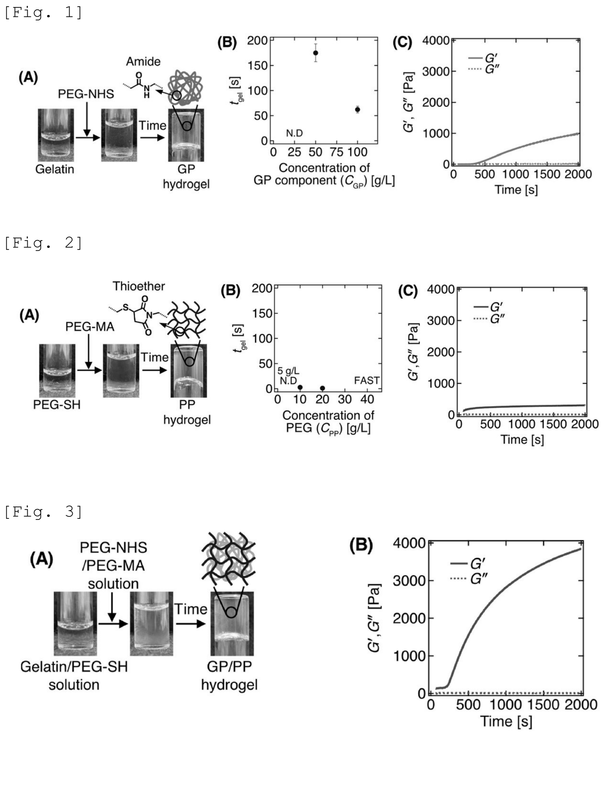

First, an 80 g/L gelatin solution and a 120 g/L PEG-NHS solution were prepared separately using neutral phosphate buffered saline (PBS) to confirm the hydrogel-forming ability of the essentially slow-solidifying GP pair. These two solutions were mixed at a volume ratio of 1 : 1 to obtain a concentration of GP component (C_{GP}) = 100 g/L. A hydrolysis-resistant amide bond was formed between gelatin and PEG-NHS, and the mixed solution solidified over time (Fig. 1(A)). The t_{gel} value was obtained by a vial inclination method while changing C_{GP} (Fig. 1(B)). It was shown that shorter t_{gel} durations can be achieved with higher C_{GP}. However, since cytotoxicity is a concern at high concentrations, a relatively low concentration (C_{GP} = 50 g/L) was selected as a representative example to alleviate this factor (Comparative Example 17). On the other hand, gelation did not occur at C_{GP} = 25 g/L in Comparative Example 18. This is probably because the concentration was too low. To obtain more quantitative results, the dynamic viscoelasticity of a representative GP hydrogel was measured (Fig. 1(C)). During the first few minutes, it was clear that the storage elastic modulus (G') is less than the loss elastic modulus (G"), indicating that it took several minutes for the material to solidify, and during this time, the material would flow down from it when being applied to the inclined surface. Thereafter, with the lapse of time, the relationship is G' > G", and gelation was confirmed.

Next, the gelation behavior of the other pair, the PP pair having a relatively short gelation time was verified (Reference Examples 1 to 4). Each PEG was prepared separately in PBS and then the two solutions were mixed to form a hydrogel similar to GP (Fig. 2(A)). Here, a thioether bond was formed between the sulfhydryl group and the maleimide group. The t_{gel} values were acquired at different concentrations (C_{PP}) as described in Reference Examples 1 to 4 of Table 1. The solidification within 5 seconds at C_{PP} ≥ 10 g/L was observed (Fig. 1E). The gelation at C_{PP} ≥ 10 g/L was difficult. Next, the dynamic viscoelasticity of a representative PP hydrogel was measured (Fig. 1F). The gelling point (that is, crossover between G' and G") was reached quickly, indicating that the crosslinking reaction was very fast at a neutral pH. Therefore, the PP hydrogel itself may be a promising carrier for delivery of GP hydrogel that is slow in hydrogel formation.

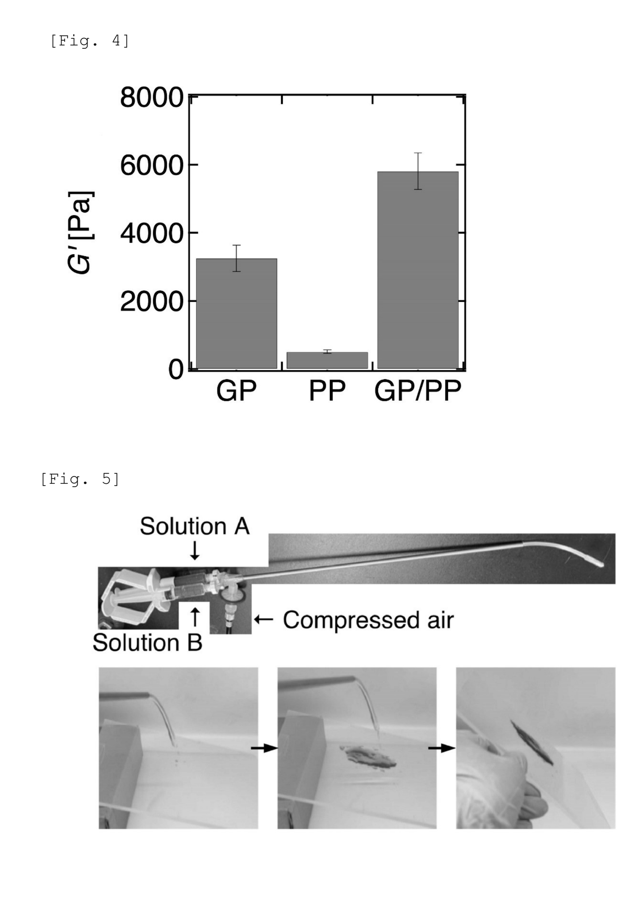

Next, instantaneous coagulation was observed when the polymer solution of Example 3 was mixed (Fig. 3(A)). When dynamic viscoelasticity measurement is performed, the relationship of G' > G" was observed immediately after mixing (Fig. 3(b)). This observed PP hydrogel-like profile indicates that crosslinking of the PP component was not inhibited by the presence of the GP component. A significant increase in G' was observed after a few minutes. Considering that this increase in G' was also observed in the GP hydrogel, it is believed that the GP network was formed over time as an interpenetrating network (IPN) within a single PEG network. The equilibrium G' value after gelation was in the order of the hydrogel derived from GP and PP components (GP/PP hydrogel) > GP hydrogel > PP hydrogel (Fig. 4).

This result means that the first and second IPNs were physically entangled, whereby the elastic modulus of the composite material increased more than the elastic modulus of each component alone. Therefore, it was suggested that these two kinds of polymer solutions solidified instantaneously when being sprayed to a predetermined place using a sprayer (Fig. 5). Therefore, by applying the polymer solution of Examples 1 to 11, the gel material can be applied without flowing out regardless of the shape of the target tissue.

### 3. Measurement of Degradation Behavior of Hydrogel

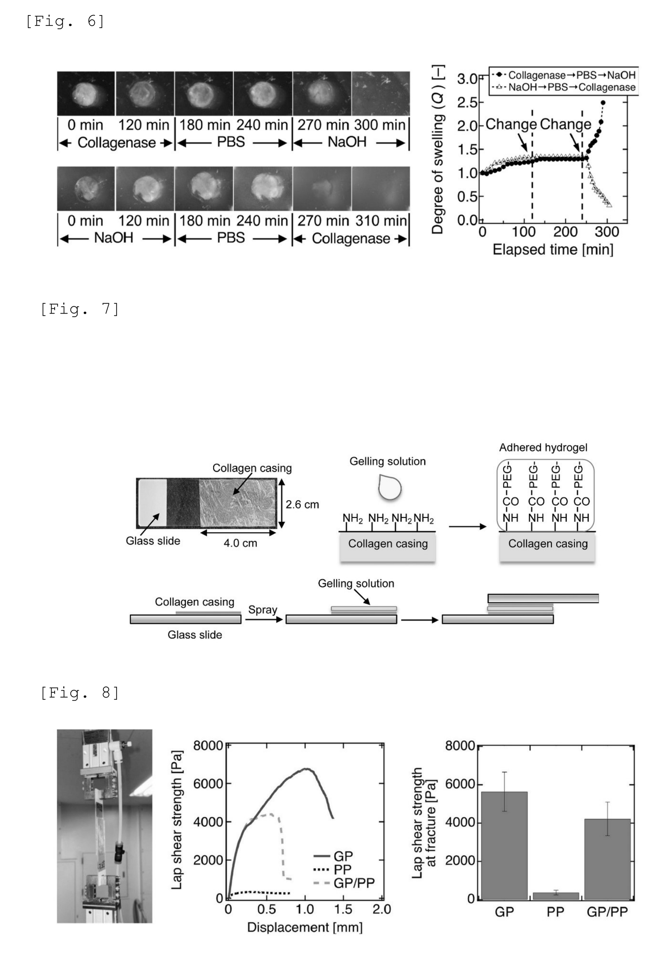

To confirm the presence of IPN, a stepwise degradation test was performed using a modified GP/PP hydrogel (hereinafter, referred to as GP/PP (ester) hydrogel) in which the PEG-MA was replaced by PEG-MA (ester) with a hydrolyzable ester moiety (Fig. 6). Before testing the GP/PP (ester) hydrogel, the GP and PP (ester) hydrogel was immersed in each of collagenase and alkaline aqueous solutions, to confirm the degradation of the hydrogel by these solutions (Fig. 6).

Next, the GP/PP (ester) hydrogel was immersed in a collagenase solution, the enzymatic reaction was stopped with neutral PBS, and the hydrogel was immersed in an alkaline aqueous solution (NaOH). As shown in the right figure of Fig. 6, in the collagenase solution, the equilibrium swelling degree (Q) increased in a time-dependent manner, but in PBS, Q was almost constant. In the next alkaline aqueous solution step, Q rapidly increased and then macroscopically disappeared. This is because when the GP network was cleaved in the first collagenase step, the balance between osmotic pressure and restoring force was lost and Q increased (that is, swelling due to decomposition).

In contrast, as shown in the right figure of Fig. 6, the PEG network derived from PP (ester) did not change in the collagenase environment, resulting in a finite degree of Q and maintained solidity as a hydrogel. In the alkaline aqueous solution, the hydrolyzable ester present in the PP (ester) network was cleaved, the balance between osmotic pressure and restoring force was lost again, and Q increased. Finally, the entire network structure of the hydrogel was lost and macroscopically disappeared.

The hydrogel was immersed in an alkaline aqueous solution, and then immersed in PBS and finally in collagenase. It was observed that Q increased with time in the alkaline aqueous solution, stable Q was observed in neutral PBS, and Q decreased rapidly in the collagenase solution, and finally the hydrogel disappeared. This is in contrast to the above hydrogel pattern in which the hydrolyzable PP (ester) network is cleaved with the first alkaline solution, the GP network maintains the hydrogel as a solid, and the subsequent collagenase solution digests gelatin enzymatically and the gel disappears. This degradation behavior with a decrease in Q is observed when cleavage of the polymer network preferentially occurs from the surface of the hydrogel due to the relatively high enzyme concentration in the outer solution. These results indicate that the GP/PP (ester) hydrogels have IPN structures derived from GP and PP (ester) networks.

These results indicate that although the polymer solution maintains a neutral pH, it can instantaneously form a hydrogel with a GP network in situ by spraying. The formed hydrogel was completely degraded when both the GP and PP networks were cleaved. The GP network was digested by collagenases ubiquitously expressed in vivo, and the PP (ester) network was hydrolyzed under physiological conditions. Therefore, the hydrogel of the present invention has biodegradability in vivo.

### 4. Evaluation of Cell Adhesiveness

In order to determine the tissue adhesion properties of the hydrogel of the present invention, the lap shear strength of the hydrogel on the collagen casing was evaluated.

A specific procedure is shown in Fig. 7. The spread-out collagen casing was previously fixed to a glass substrate with a cyanoacrylate adhesive. Two identical substrates with a collagen casing were prepared and a hydrogel was applied between the collagen casings. Next, the test piece was pulled with a tensile tester, the lap shear strength versus displacement was acquired, and the lap shear strength at fracture was calculated (Fig. 8).

Here, the lap shear strength is expressed as F/Sᵢₙᵢ, where F is the given force and Sᵢₙᵢ is the initial adhesion area of 10.4 cm². The lap shear strength at fracture is expressed as Fₘₐₓ/Sᵢₙᵢ, where Fₘₐₓ is the maximum force given and Sᵢₙᵢ is 10.4 cm².

As shown in the right figure of Fig. 8, the PP hydrogel did not show a significant lap shear strength, due to the lack of cysteine residues (that is, a maleimide reactive sulfhydryl group) in the animal-derived collagen and the reaction between the maleimide group and the amino group being effective only under alkaline conditions. In contrast to the PP hydrogel, in the two groups including the GP hydrogel, a significant increase in lap shear strength with respect to displacement was observed. This strongly suggests that the NHS group reacted with gelatin to form a polymer network within the GP hydrogel and formed a chemical bond with the amino group on the surface of the collagen casing. This result is consistent with previous studies of identifying hydrogels composed of gelatin and PEG-NHS that adhere to biological tissues. The GP/PP hydrogel of the present invention showed a lap shear strength similar to that of the GP hydrogel. Considering that adhesion was not observed in the PP hydrogel, it is considered that a part of the PP hydrogel formed a crosslink with the collagen casing of the GP/PP hydrogel. These results indicate that by forming both the GP hydrogel and the PP hydrogel, both the instantaneous coagulation properties derived from the PP hydrogel and the tissue adhesion properties derived from the GP hydrogel are obtained.

### 5. Encapsulation of Cells in Hydrogel

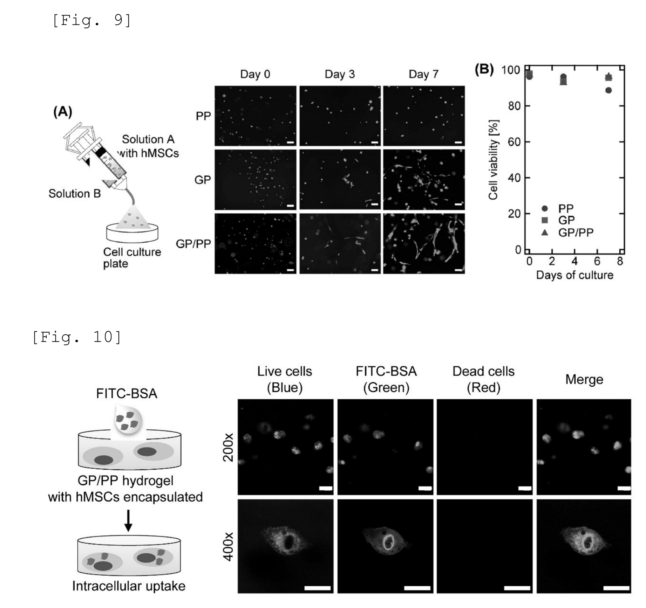

In order to evaluate the cell compatibility of the hydrogel of the present invention, human mesenchymal stem cells (hMSC) were encapsulated in the hydrogel and cultured in a normal medium, and the morphology of the cells was observed (Fig. 9(A)). The proportion of living cells was calculated from the obtained fluorescence image (Fig. 9(B)). A specific experimental procedure is as follows.

### <Cell Culture with Hydrogel Formed by Spraying>

hMSCs (passage 2) were cultured using a 10 cm dish (Corning Inc., Corning, NY, USA) in 10 mL DMEM (10% FBS and 1% PS) at 37°C in a 5% CO₂ atmosphere for 1 week. The medium was changed every 2 to 3 days to ensure nutrient supply. The polymer solutions A and B were sterilized using a 0.22 µm filter and a sprayer (MIS Spray Applicator, Medmix AG, Baar, Switzerland) was sanitized with 70% ethanol and then washed with DMEM. hMSCs (passage 3) were suspended in the polymer solution A at a cell density of 5.0 × 10⁵ cells/mL. The solutions A and B with cells suspended were separately filled into 5 mL syringes and sprayed into each well of a 12-well cell culture plate. The sprayed solution was incubated at 37°C for 30 minutes under 5% CO₂ to obtain a hydrogel. The final hydrogel volume and cell number were 1 mL and 2.5 × 10⁵ cells, respectively. The prepared hydrogel was immersed in 2 mL of DMEM and incubated for 1 week. The medium was changed every 2 to 3 days.

### <Observation of Cell Morphology and Viability>

DMEM containing 1 µM of Calcein AM and 2 µM of ethidium homodimer was prepared according to manufacturer's protocol. hMSCs were incubated in the hydrogel for 0, 4, and 7 days, followed by further incubation in DMEM prepared here for 10 minutes at 37°C and 5% CO₂ from each time point. Next, the hydrogel was washed three times with PBS. The cells encapsulated in the hydrogel were observed with a fluorescence microscope, the number of living and dead cells was counted using Image J software, and the cell viability was calculated as the ratio of living cells to all cells.

As shown in Fig. 9, in the case of the cell-encapsulated GP hydrogel, all the cells were spherical on Day 0, but some cells were elongated on Day 3, and the proportion of elongated cells further increased on Day 7. A high cell viability (> 90%) indicates that the cells were not affected by the shear stress due to spraying. Morphological changes and cell viability were similar to those of previously reported gelatin and the PEG hydrogel, confirming the validity of this cell culture protocol.

As a result of verification for the cell-encapsulated PP hydrogel in the same culture system, similarly to the GP hydrogel, the cells were spherical immediately after preparation, but unlike the GP hydrogel, they remained spherical until Day 7. A slight decrease in cell viability was also observed. This may be due to anoikis (apoptotic cell death caused by insufficient cell-matrix interactions), as hMSCs cannot adhere or extend in pure PEG hydrogels. On the other hand, the cell viability exceeded 80%, and the crosslinking reaction of the PP hydrogel was not highly cytotoxic, suggesting that it is promising as a carrier material for the GP hydrogel.

The cell viability of the GP/PP (ester) hydrogel of the present invention can be comparable to the cell viability (> 90%) of the GP hydrogel alone, the morphology of the cells at Day 7 was similar to that of the GP hydrogel, and in both cases, some cells were elongated. These results indicate that the cell compatibility and cell adhesiveness of the GP hydrogel are maintained even when the PP hydrogel component is included.

Next, the material permeability of the GP/PP (ester) hydrogel was evaluated. In this assay, bovine serum albumin (BSA) labeled with fluorescein isothiocyanate (FITC) was added to the medium (that is, the outer side of the hydrogel) (left figure in Fig. 10). This is because it is known that this compound passes through a cell membrane and enters cytoplasm by endocytosis. A specific procedure is as follows.

DMEM containing 2 µM of Calcein Blue and 2 µM of PI was prepared. FITC-BSA was added to the prepared DMEM at 0.1 g/L. A cell-encapsulated GP/PP hydrogel was prepared and incubated in DMEM containing FITC-BSA for 30 minutes, and then incubated in DMEM containing Calcein Blue and PI for 10 minutes at 37°C under 5% CO₂. Next, the hydrogel was washed three times with PBS and observed by CLSM.

The obtained results are shown in the right figure of Fig. 10. After addition of BSA, the cytoplasm turned green in the cross-sectional image of the cells obtained with a confocal microscope. This indicates that BSA diffused into the hydrogel and reached the cytoplasm of encapsulated cells. Since BSA is a protein having a molecular weight of about 66 kDa, it is considered that other proteins having a similar molecular weight or a lower molecular weight can freely pass through the hydrogel. Such excellent permeability is important for cell medicine because not only cell survival but also therapeutic effects are obtained from proteins secreted by cells such as hMSCs.

The above results demonstrate that a hydrogel containing two components: 1) a tissue-adhesive gelatin/PEG hydrogel and 2) an instantaneously solidifying PEG hydrogel can be instantaneously solidified by spraying and exhibits excellent cell adhesiveness. Human mesenchymal stem cells (hMSC) can be encapsulated in the hydrogel of the present invention, and excellent cell viability (> 90%) in the hydrogel was exhibited. The hydrogel was shown to be permeable to proteins. That is, it was demonstrated that the hydrogel of the present invention can immobilize cells that secrete beneficial proteins for various biological tissues, and has a possibility of becoming a basic tool in the field of cellular medicine.

## Claims

1. A hydrogel comprising: a first polymer network having a structure in which two or more polymers (PEG-A) having a polyethylene glycol backbone are crosslinked with each other; and a second polymer network having a structure in which a polypeptide and a polymer (PEG-B) having a polyethylene glycol backbone are crosslinked with each other.

2. The hydrogel according to claim 1, wherein the hydrogel has a gelation time within a range of 10 seconds, and wherein the gelation time is a time required until a storage elastic modulus G' and a loss elastic modulus G" satisfy G' = G".

3. The hydrogel according to claim 1, wherein a concentration of the polypeptide in the second polymer network is 10 g/L or more and 120 g/L or less in the hydrogel.

4. The hydrogel according to claim 1, wherein a total content of the polymers having a polyethylene glycol backbone (PEG-A and PEG-B) in the hydrogel is 15 g/L or more.

5. The hydrogel according to claim 1, wherein the first polymer network has a cleavable moiety selected from the group consisting of an ester bond, a thioester bond, a disulfide bond, a phosphate ester bond, a sulfonate ester bond, and a combination thereof.

6. The hydrogel according to claim 1, wherein the polymers (PEG-A) having a polyethylene glycol backbone in the first polymer network comprise a first polymer having two or more nucleophilic functional groups in total at a side chain or a terminal and a second polymer having two or more electrophilic functional groups in total at a side chain or at a terminal.

7. The hydrogel according to claim 1, wherein the polymer (PEG-B) having a polyethylene glycol backbone in the second polymer network has two or more electrophilic functional groups capable of reacting with an amino group present in a molecule of the polypeptide to form an amide bond or a urethane bond at a side chain or a terminal.

8. The hydrogel according to claim 1, wherein the polypeptide is one or more selected from the group consisting of gelatin, collagen, albumin, laminin, fibrinogen, elastin, fibronectin, or a derivative thereof.

9. The hydrogel according to claim 1, wherein a concentration of the polypeptide in the hydrogel is 10 g/L or more, and a concentration of the polymer (PEG-B) having a polyethylene glycol backbone in the second polymer network is 15 g/L or more.

10. The hydrogel according to claim 1, wherein a pH of a solvent contained in the hydrogel is in a range of 6.5 to 11.5.

11. A scaffold material for cell adhesion, comprising the hydrogel according to any one of claims 1 to 10.

12. A gel composition comprising cells supported in the hydrogel according to any one of claims 1 to 10.

13. The gel composition according to claim 12, wherein the cells are somatic stem cells.

14. A kit for rapidly preparing a hydrogel, comprising:
a solution X containing polymers A and B and a solution Y containing a polypeptide and a polymer C separately stored, wherein
the polymer A is a polymer having a polyethylene glycol backbone chemically crosslinkable with the polypeptide,
the polymers B and C are a combination of polymers having a polyethylene glycol backbone chemically crosslinkable with each other, and
a gelation time of the hydrogel obtained by mixing the solutions X and Y is within 10 seconds, the gelation time being a time required until a storage elastic modulus G' and a loss elastic modulus G" satisfy G' = G".

15. The kit according to claim 14, wherein the polymer A has two or more electrophilic functional groups capable of reacting with an amino group present in a molecule of the polypeptide to form an amide bond or a urethane bond at a side chain or a terminal.

16. The kit according to claim 14, wherein the polymers B and C are a combination of a polymer having two or more nucleophilic functional groups in total at a side chain or a terminal and a polymer having two or more electrophilic functional groups in total at a side chain or a terminal.

17. The kit according to claim 14, wherein the polypeptide in the solution Y is one or more selected from the group consisting of gelatin, collagen, albumin, laminin, fibrinogen, elastin, fibronectin, or a derivative thereof.

18. The kit according to claim 14, wherein a reaction rate of the polymer B and the polymer C is in a range of 60% to 100%.

19. The kit according to claim 14, which is used for spraying the solutions X and Y onto a predetermined area by a sprayer.

20. The kit according to claim 14, wherein the solutions X and Y are each stored in separate sprayers.
